(19) Europäisches Patentamt
European Patent Office
Office européen des brevets

(11) Veröffentlichungsnummer: **0 210 574 B1**

(12) # EUROPÄISCHE PATENTSCHRIFT

(45) Veröffentlichungstag der Patentschrift: **23.10.91**

(51) Int. Cl.5: **C07C 229/56**, C07C 205/57, C07D 307/52, A61K 31/195, A61K 31/34

(21) Anmeldenummer: **86110027.9**

(22) Anmeldetag: **22.07.86**

Die Akte enthält technische Angaben, die nach dem Eingang der Anmeldung eingereicht wurden und die nicht in dieser Patentschrift enthalten sind.

(54) **N-Substituierte 5-Nitroanthranilsäuren, Verfahren zu ihrer Herstellung, ihre Verwendung sowie pharmazeutische Präparate auf Basis dieser Verbindungen.**

(30) Priorität: **31.07.85 DE 3527409**
**05.08.85 DE 3528048**

(43) Veröffentlichungstag der Anmeldung:
**04.02.87 Patentblatt 87/06**

(45) Bekanntmachung des Hinweises auf die Patenterteilung:
**23.10.91 Patentblatt 91/43**

(84) Benannte Vertragsstaaten:
**AT BE CH DE FR GB IT LI LU NL SE**

(56) Entgegenhaltungen:
**EP-A- 0 023 569**
**GB-A- 2 019 214**

**CHEMICAL ABSTRACTS, Band 95, Nr. 5, 3. August 1981, Seite 721, Zusammenfassung Nr. 42698a, Columbus, Ohio, US; & JP-A-80 145 648**

(73) Patentinhaber: **HOECHST AKTIENGESELLSCHAFT**
**Postfach 80 03 20**
**W-6230 Frankfurt am Main 80(DE)**

(72) Erfinder: **Greger, Rainer, Prof. Dr.**
**Finkenstrasse 37**
**W-6056 Heusenstamm(DE)**
Erfinder: **Englert, Heinrich Christian, Dr.**
**Stormstrasse 13**
**W-6238 Hofheim am Taunus(DE)**
Erfinder: **Lang, Hans-Jochen, Dr.**
**Rüdesheimer Strasse 7**
**W-6238 Hofheim am Taunus(DE)**
Erfinder: **Hropot, Max, Dr.**
**Friedrich-Stolz-Strasse 13**
**W-6093 Flörsheim am Main(DE)**

EP 0 210 574 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft 5-Nitroanthranilsäuren der Formel I

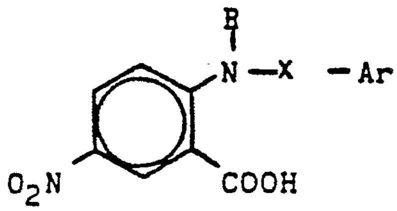

I

in welcher

R für Wasserstoff steht

X für eine Kette $(CH_2)_n$ steht, wobei $n = 1,2,3$ oder 4 ist und wobei die Kette unsubstituiert oder durch 1 bis 2n Alkylreste mit 1-2 C-Atomen oder zusätzlich durch den Rest Ar, wobei Ar wie unten definiert ist, substituiert ist,

Ar für ein aromatisches oder heteroaromatisches System steht, das gegebenenfalls durch 1 bis 3 gleiche oder verschiedene Reste ($C_1$ oder $C_2$)-Alkyl, ($C_1$ oder $C_2$)-Alkoxy, Halogen oder Trifluormethyl substituiert sein kann,

sowie deren pharmazeutisch verträgliche Salze.

Unter einem aromatischen System Ar wird vorzugsweise Phenyl verstanden, ein 5- oder 6-gliedriges heteroaromatisches System Ar ist vorzugsweise ein Rest eines 5- oder 6-gliedrigen O-, N-, und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl,Pyrimidinyl, Pyridazinyl, Triazinyl. Unter Halogen wird F, Cl, Br, oder J vorzugsweise Cl oder Br verstanden.

Falls Verbindungen der Formel I chirale C-Atome aufweisen, so sind sowohl am jeweiligen Zentrum S-konfigurierte als auch R-konfigurierte Verbindungen Gegenstand der Erfindung. Die Verbindungen können dann als optische Isomere als Diastereoisomere, als Racemate oder als Gemische derselben vorliegen.

Den Verbindungen der Formel I ähnliche Verbindungen, die anstelle der $NO_2$-Gruppe andere funktionelle Gruppen in den verschiedenen Positionen des Benzolkerns tragen, sind bekannt. Pharmakologische Wirkungen wurden beispielsweise für die 2-Chlor-6-((2-Phenethyl)amino)-benzoesäure (hypoglykaemisch; Chem. Pharm. Bull., 27(2), 522-7, + 1468-72), die N-Phenethylanthranilsäure (antiphlogistisch; Indian J. Med. Res., 78(Juli), 147-50) sowie für die 5-(Aminosulfonyl)-2-((2-Phenylethyl)amino)-4-(Phenylmethyl)-benzoesäure (diuretisch, J. Med. Chem., 16(2), 127-30) berichtet.

Es war daher äußerst überraschend, daß die Verbindungen der Formel I eine abschwellende Wirkung auf Gehirnoedeme aufweisen, ohne daß dies von einer diuretischen Wirkung begleitet wird.

Weiterhin war es überraschend, daß einige der Verbindungen auch eine intestinale, insbesondere eine diarrhoische oder antidiarrhoische Wirkung aufweisen.

Bevorzugt sind Verbindungen der Formel I, die dadurch gekennzeichnet sind, daß R für Wasserstoff, X für $(CH_2)_n$ mit $n = 2,3$ und Ar für den unsubstituierten Phenylrest steht.

Die Erfindung betrifft weiterhin ein Verfahren zur Herstellung von 5-Nitroanthranilsäuren der Formel I, das dadurch gekennzeichnet ist, daß man

A) Verbindungen der Formel II,

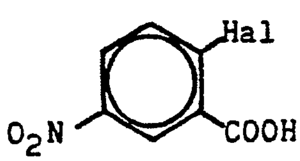

II

bei der Hal für Cl, F, und Br steht, umsetzt mit Aminen der allgemeinen Formel Ar-X-NRH, wobei X, R, und Ar die oben erwähnten Bedeutungen haben,

B) Verbindungen der Formel III,

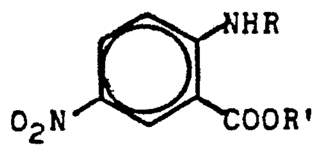

III

in der R die oben festgelegte Bedeutung hat und in der für R' Alkyl mit 1-4 C-Atomen steht, umsetzt mit Verbindungen der Formel Z-X-Ar, in der X und Ar die oben festgelegten Bedeutungen aufweisen und Z für eine Fluchtgruppe steht, und den Rest R' mit bekannten Methoden abspaltet,

C) Verbindungen der Formel III, in der R und R' die oben genannten Bedeutungen haben, R' jedoch zusätzlich für Wasserstoff steht, umsetzt mit Verbindungen der Formel Ar-X'-COHal, wobei Ar die oben genannte Bedeutung hat und X' für eine Kette $(CH_2)_{n-1}$ steht, die wie oben definiert durch 1 bis (2n-1) Alkylreste und/oder durch den Rest Ar substituiert sein kann und in der Hal für Chlor oder Brom steht, zu Verbindungen der Formel IV

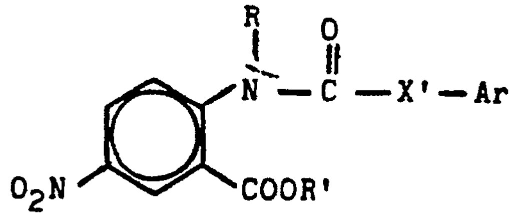

IV

und diese reduziert zu Verbindungen der Formel V

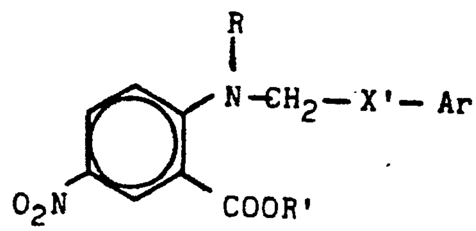

V

und den Rest R' abspaltet.

Die nach den Varianten A-C erhaltenen Verbindungen der Formel I werden gegebenenfalls in ihre pharmazeutisch verträglichen Salze überführt.

Als Fluchtgruppe Z der Verfahrensvariante B) sind alle leicht nukleophil verdrängbaren Gruppen wie beispielsweise Halogen, Tosylat, Mesylat, Trifluormethansulfonat, Acetoxy oder Trifluoracetoxy geeignet.

Werden Verbindungen der allgemeinen Formel I nach Verfahrensvariante A) hergestellt, so geschieht dies vorteilhaft dadurch, daß man die Verbindungen der Formel II mit den Aminen RNH-X-Ar unter Zusatz eines Säurefängers umsetzt. Es kommen prinzipiell alle basischen Stoffe in Frage, die das bei der Reaktion entstehende HHal zu binden vermögen, beispielsweise Kaliumcarbonat, Natriumhydroxid, Natriumhydrogencarbonat, Triethylamin oder etwa ein Überschuß des Amins RNH-X-Ar. Unter Umständen kann es von Vorteil sein, die Reaktion zusätzlich nach Art der Ullmannreaktion durch den Zusatz von Cu-Pulver oder Cu-Bronze zu katalysieren. Als Lösungsmittel kommen dabei alle den Reaktionspartnern gegenüber inerte Lösemittel wie z.B. Dimethylacetamid oder Ethanol in Frage. Bei Verwendung flüssiger Amine RNH-X-Ar kann mitunter ganz auf ein Lösemittel verzichtet werden.

Verfahrensvariante B) umfaßt Alkylierung der aromatischen Amine III mit den Alkylierungsmitteln Z-X-Ar. Dies wird in an sich bekannter Weise so durchgeführt, daß man die Reaktionspartner, gegebenenfalls unter Zusatz eines Säurefängers, in einem inerten Lösemittel zur Reaktion bringt. Als besonders geeignet haben sich dabei dipolar-aprotische Lösemittel erwiesen wie etwa Dimethylformamid oder Dioxan. Als Säurefänger kommen alle basischen Stoffe in Frage, die die bei der Reaktion freiwerdende Säure zu binden vermögen, beispielsweise die unter Verfahrensvariante A) genannten Basen. Als besonders vorteilhaft hat sich dabei die Verwendung von Kaliumkarbonat in Dioxan herausgestellt. I. A. sind erhöhte Reaktionstemperaturen zur Erzielung befriedigender Umsätze von Vorteil, beispielsweise Rückflußtemperatur von Dioxan. Bei den

solchermassen erhaltenen Verbindungen der Formel I ist die COOH-Gruppe noch durch R'-OH verestert. Durch Säure- oder basenkatalysierte Hydrolyse lassen sich diese Verbindungen leicht nach Standardmethoden in die freien Anthranilsäuren umwandeln.

Werden Verbindungen der Formel I nach Verfahrensvariante C) hergestellt, so werden zunächst Anthranilsäureester der Formel III acyliert durch die Säurehalogenide Ar-X'-Hal. Die Ausführung orientiert sich an Standardmethoden für derartige Acylierungsreaktionen, beispielsweise durch die Verwendung von Triethylamin oder Pyridin als Säurefänger und von den Reaktionspartnern gegenüber neutralen Lösemitteln wie Ethylacetat oder Dioxan. Die anschließende Reduktion der Amide IV zu den Aminen V kann ebenfalls nach Standardmethoden durchgeführt werden. Als besonders vorteilhaft hat sich dabei die Verwendung eines Reduktionsmittels wie etwa Natriumborhydrid in Gegenwart eine- Lewissäure wie Bortrifluorid erwiesen. Die dadurch erhaltenen Verbindungen V werden durch Standardmethoden verseift zu den Säuren der Formel I.

Die erfindungsgemäßen Verbindungen der Formel I sowie deren pharmazeutisch verträgliche Salze sind Mittel, die der Entstehung eines Gehirnödems entgegenwirken oder dessen Abschwellung herbeiführen oder beschleunigen können. Sie werden in Dosierungen von mindestens 0,01 mg/kg, vorzugsweise 0,05 mg/kg und insbesondere 0,5 mg/kg bis maximal 100 mg/kg, vorzugsweise 50 mg/kg und insbesondere 20 mg/kg in Kapseln, Dragees, Tabletten oder Lösungen mit verschiedenen Zusätzen enteral z.B. oral oder parenteral (wie etwa Injektion in das Gefäßsystem, z.B. intravenös) verabfolgt. Sie eignen sich zur Behandlung von Hirnödemen unterschiedlicher Pathogenese, beispielsweise solcher Ödeme, die durch Schädeltraumen infolge stumpfer Verletzungen entstehen aber auch ischämisch bedingte Hirnödeme wie etwa nach Apoplex oder nach epileptischen Anfällen oder wie sie in Begleitung gewisser Gehirntumoren entstehen, können behandelt werden.

Beispiel 1 5-Nitro-2-(3-phenylpropylamino)-benzoesäure

Eine Lösung aus 15,3 g 2-Chlor-5-nitrobenzoesäure und 49 g 3-Phenylpropylamin in 60 ml Dimethylacetamid wird über 2 Stunden auf 145 °C erhitzt. Nach dem Abkühlen versetzt man mit 300 ml Wasser und stellt mit 2 n HCl sauer (pH 2 bis 1).
Der gelbe kristalline Niederschlag wird abfiltriert, mehrmals mit Wasser gewaschen und nach dem Trocknen aus Isopropanol umkristallisiert.
Gelbe Kristalle, Schmp. 178 - 180 °C.

Beispiel 2 5-Nitro-2-(2-phenylethyl-amino)-benzoesäure

Eine Lösung von 5,6 g 2-Fluor-5-nitrobenzoesäure und 14,5, g 2-Phenylethylamin in 20 ml Dimethylacetamid wird analog Beispiel 1 umgesetzt und aufgearbeitet. Gelbe Kristalle aus Isopropanol, Schmp. 116 - 118 °C.

Analog der in Beispiel 1 und 2 angegebenen Vorschrift lassen sich unter Verwendung von 2-Chlor- bzw. 2-Fluor-5-nitrobenzoesäure und des entsprechenden Amins die nachfolgend aufgeführten erfindungsgemäßen Verbindungen herstellen:

| | |
|---|---|
| Beispiel 3 | 2-(2,2-Diphenylethylamino)-5-nitrobenzoesäure,<br>Schmp. 232 - 234 °C. |
| Beispiel 4 | 2-(4-Phenylbutylamino)-5-nitrobenzoesäure,<br>Schmp. 180 - 182 °C. |
| Beispiel 5 | 2-(1-Methyl-3-phenylpropylamino)-5-nitrobenzoesäure,<br>Schmp. 228 - 230 °C. |
| Beispiel 6 | 2-(3,3-Diphenylpropylamino)-5-nitrobenzoesäure,<br>Schmp. 203 °C. |
| Beispiel 7 | 5-Nitro-2-(1-phenylethylamino)-benzoesäure<br>(Racemat und optische Isomere) |
| Beispiel 8 | 2-(3,4,5-Trimethoxybenzylamino)-5-nitrobenzoesäure, |
| Beispiel 9 | 2-[2-(2-Methoxyphenyl-ethyl)-amino]-5-nitrobenzoesäure |
| Beispiel 10 | 2-[2-(3,4-Dimethoxyphenyl-ethyl)-amino]-5-nitrobenzoesäure |
| Beispiel 11 | 2-[2-(4-Methoxyphenyl-ethyl)-amino)-5-nitrobenzoesäure |
| Beispiel 12 | 2-[2-(2-Chlorphenyl-ethyl)-amino]-5-nitrobenzoesäure |
| Beispiel 13 | 2-[2-(4-Chlorphenyl-ethyl)-amino]-5-nitrobenzoesäure |
| Beispiel 14 | 2-[2-(3-Trifluormethylphenyl-ethyl)-amino]-5-nitrobenzoesäure |
| Beispiel 15 | 2-(2-Furylmethylamino)-5-nitrobenzoesäure |

Eine Lösung von 14,7 g 2-Fluor-5-nitrobenzoesäure und 23,1 g 2-Furylmethylamin in 250 ml Ethanol wird über 6 Stunden am Rückflußkühler gekocht. Nach Abdestillieren des Lösungsmittels unter vermindertem Druck löst man den Rückstand in 150 ml Wasser, stellt mit 2 n HCl auf etwa pH 2 und filtriert den Niederschlag ab. Nach mehrmaligem Waschen des Niederschlages mit Wasser trocknet man im Luftstrom.
Hellgelbe Kristalle,
Schmp. 190 - 192 °C (aus Isopropanol).

Beispiel 16 2-Benzylamino-5-nitrobenzoesäure

Pharmakologische Daten

Hirnödem

(Bilaterale temporäre Gehirnischämie beim mongolischen Gerbil).

Methode:

Die Versuche werden an männlichen mongolischen Gerbils (Meriones unguiculatus; Stamm: Kastengrund, Hoechst AG, Frankfurt am Main) mit einem Körpergewicht von 50 - 70 g in Narkose durchgeführt. Für die Narkose wird ein tragbares Narkosegerät Halothanvapor 19.1 (Drägerwerk AG, Lübeck-West-Deutschland) verwendet. Einstellung der Narkosegase: $O_2$ 1.1 l/min + $N_2$ 2.0 l/min + Halothan 3.2 Vol.% bis zum Erreichen des Toleranzstadiums. Das Atemgas Halothangemisch wird in einen kleinen Exsikkator geleitet, in dem die Tiere bis zum Erreichen des Toleranzstadiums verbleiben. Anschließend werden die Tiere aus dem Exsikkator entfernt, auf einen Rattenoperationstisch fixiert und die Narkose über eine Atemmaske bei einer Halothankonzentration von 1.2 Vol.% oder geringer fortgesetzt. Nun werden die beiden A. carotides communes schonend freigelegt und mit dem Microgefäßclips für 20 min. verschlossen. Danach erfolgt eine Rezirkulation für 90 min. Nach Verschluß der Gefäße werden die Tiere in Beobachtungsgläser gesetzt, die mit Hilfe von Heizkissen auf eine Bodentemperatur von ca. 29 °C gebracht werden. Die Beobachtung der Tiere in den Glasgefäßen erstreckt sich über 110 min., dabei werden die neurologischen Symptome entsprechend einer Klassifizierung festgehalten.
Die Testsubstanzen werden vor dem Gefäßverschluß in einem Injektionsvolumen von 5 ml/kg Körpergewicht oral verabreicht. Die Kontrolltiere erhalten das entsprechende Vehikel in gleichem Volumen. Nach einer Beobachtungszeit von 110 min. werden die Tiere in Narkose dekapitiert und die Gehirne entnommen. Das Kleinhirn wird verworfen und das Feuchtgewicht der beiden Gehirnhälften bestimmt.

Die gewogenen Gehirne werden in einem Trockenschrank bei 95 °C 2 Tage getrocknet. Nach der Bestimmung des Trockengewichtes wird der Hirnödemgrad in Prozent des Wassergehaltes errechnet.

Literatur:

1. Levy, D.E. and T.E. Duffy: J. Neurochem. 28:63 , 1977
2. Welsh, F.A. and W. Rieder: J. Neurochem. 31:299, 1978
3. Cain, A.P. and M.E. Corcoran : Electroenceph. Clin. Neurophys. 49 : 360, 1980

Wirkung in einer Dosis von 100 mg/kg oral auf Hirnödem am Gerbil.

| Präparat: | Dosis: | Wassergehalt des Gehirnes % $\bar{x} \pm SD$ |
|---|---|---|
| Scheinoperierte Kontrolle (n=5) | Vehikel (2 Vol%ige Stärkelösung) | 79.59 ± 0.11 |
| Hirnödem-Kontrolle (n=5) | Vehikel | 80.60 ± 0.06 |
| 5-Nitro-2-(3-phenylpropylamino)-benzoesäure (n=6) | 100 mg/kg p.o. (aufgeschlämmt im Vehikel) | 80.45 ± 0.07 |

SD = standard deviation

## Patentansprüche

**Patentanspüche für folgende Vertragsstaaten: BE, CH, DE, FR, GB, IT, LI, LU, NL, SE**

**1.** Verbindungen der Formel I

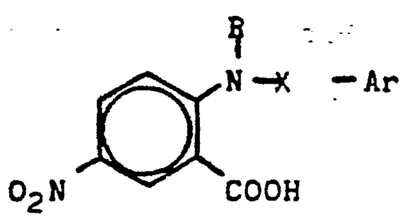

I

in welcher

R für Wasserstoff steht,

X für eine Kette $(CH_2)_n$ steht, wobei n 1,2,3 oder 4 ist, und welche Kette unsubstituiert oder durch einen bis 2n $(C_1-C_2)$-Alkylreste substituiert ist, wobei die Kette zusätzlich durch einen Rest Ar substituiert sein kann, der wie unten definiert ist,

Ar für ein aromatisches oder heteroaromatisches System steht, das unsubstituiert oder durch 1 bis 3 gleiche oder verschiedene Gruppen $(C_1-C_2)$-Alkyl, $(C_1-C_2)$-Alkoxy, F, Cl, Br, J oder $CF_3$ substituiert ist,

sowie deren pharmazeutisch verträgliche Salze.

**2.** Verbindung der Formel I nach Anspruch 1, dadurch gekennzeichnet, daß

X für $(CH_2)_n$ mit n = 2,3 und

Ar für den unsubstituierten Phenylrest steht.

**3.** Verfahren zur Herstellung einer Verbindung I nach Anspruch 1, dadurch gekennzeichnet, daß man

a) eine Verbindung der Formel II

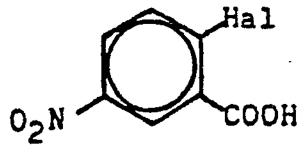

II

in der Hal für F, Cl oder Br steht, umsetzt mit Aminen der Formel

Ar-X-NHR,

worin Ar, X und R die genannten Bedeutungen haben,
b) eine Verbindung der Formel III

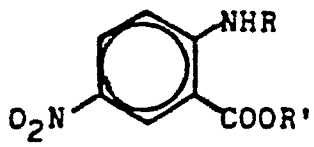

III

in der R wie oben definiert ist und in der R' für ($C_1$-$C_4$)-Alkyl steht, umsetzt mit einer Verbindung der Formel

Z-X-Ar,

in der X und Ar wie oben definiert sind, und in der Z für eine Fluchtgruppe steht, und daß man den Rest R' abspaltet,
c) eine Verbindung der Formel III

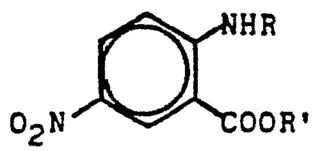

III

in der
R und R' die obengenannten Bedeutungen haben, R' jedoch zusätzlich für Wasserstoff steht, umsetzt mit Verbindungen der Formel

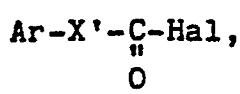

worin
Ar die obengenannte Bedeutung hat und X' für eine Kette $(CH_2)_{n-1}$ steht, die unsubstituiert oder durch 1 bis (2n-1)Alkylreste mit 1 bis 2 C-Atomen oder durch einen zusätzlichen Rest Ar substituiert ist,
und
Hal für Cl oder Br steht,
zu einer Verbindung der Formel IV

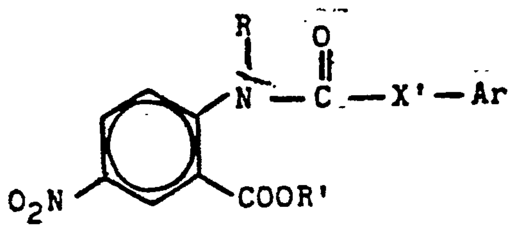

IV

und diese zu einer Verbindung V

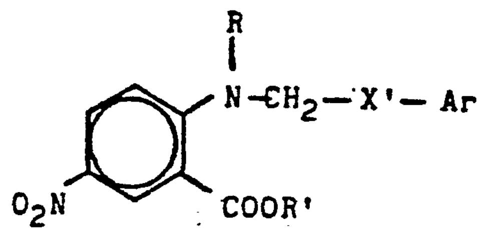

V

reduziert
und den Rest R' abspaltet.

4. Medikament, dadurch gekennzeichnet, daß es eine wirksame Menge mindestens einer Verbindung I nach Anspruch 1 enthält.

5. Verwendung einer Verbindung I nach Anspruch 1 zur Herstellung eines Medikaments zur Behandlung des Gehirnödems.

6. Verwendung einer Verbindung I nach Anspruch 1 zur Herstellung eines Medikaments zur Behandlung der Diarrhoe.

**Patentansprüche für folgende Vertragsstaaten: AT**

1. Verfahren zur Herstellung einer Verbindung I

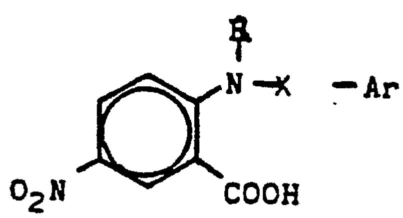

I

in welcher

- R für Wasserstoff steht,
- X für eine Kette $(CH_2)_n$ steht, wobei n 1,2,3 oder 4 ist, und welche Kette unsubstituiert oder durch einen bis 2n $(C_1-C_2)$-Alkylreste substituiert ist, wobei die Kette zusätzlich durch einen Rest Ar substituiert sein kann, der wie unten definiert ist,
- Ar für ein aromatisches oder heteroaromatisches System steht, das unsubstituiert oder durch 1 bis 3 gleiche oder verschiedene Gruppen $(C_1-C_2)$-Alkyl, $(C_1-C_2)$-Alkoxy, F, Cl, Br, J oder $CF_3$ substituiert ist,

sowie deren pharmazeutisch verträgliche Salze,
dadurch gekennzeichnet, daß man
a) eine Verbindung der Formel II

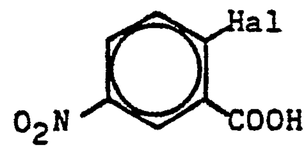

II

in der Hal für F, Cl oder Br steht, umsetzt mit Aminen der Formel

Ar-X-NHR,

worin Ar, X und R die genannten Bedeutungen haben,
b) eine Verbindung der Formel III

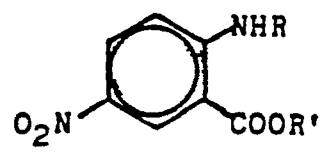

III

in der R wie oben definiert ist und in der R' für ($C_1$-$C_4$)-Alkyl steht,
umsetzt mit einer Verbindung der Formel

Z-X-Ar,

in der X und Ar wie oben definiert sind, und in der Z für eine Fluchtgruppe steht,
und daß man den Rest R' abspaltet,
c) eine Verbindung der Formel III

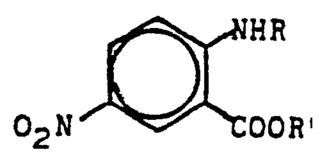

III

in der
R und R' die obengenannten Bedeutungen haben, R' jedoch zusätzlich für Wasserstoff steht,
umsetzt mit Verbindungen der Formel

$$Ar-X'-\underset{\underset{O}{\|}}{C}-Hal,$$

worin
Ar die obengenannte Bedeutung hat und X' für eine Kette $(CH_2)_{n-1}$ steht, die unsubstituiert oder durch 1 bis (2n-1)Alkylreste mit 1 bis 2 C-Atomen oder durch einen zusätzlichen Rest Ar substituiert ist,
und
Hal für Cl oder Br steht,
zu einer Verbindung der Formel IV.

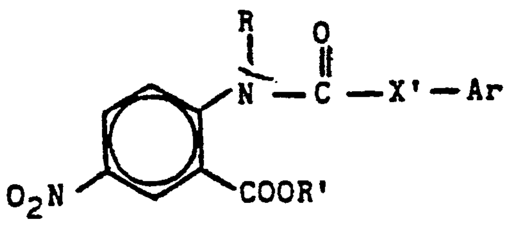

IV

und diese zu einer Verbindung V

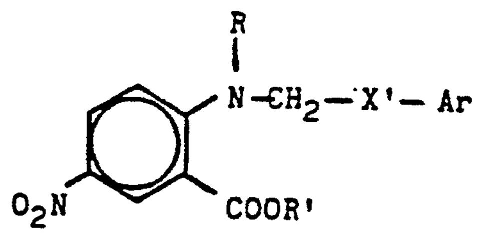

V

reduziert und den Rest R' abspaltet.

2. Verfahren nach Anspruch 1, dadurch gekennzeichnet, daß
   X für $(CH_2)_n$ mit n = 2, 3 und
   Ar für den unsubstituierten Phenylrest steht.

3. Verfahren zum Herstellen eines Medikaments zur Behandlung des Gehirnödems, dadurch gekennzeichnet, daß man eine Verbindung I, hergestellt nach Anspruch 1, mit pharmazeutisch geeigneten Hilfsstoffen mischt.

4. Verfahren zum Herstellen eines Medikaments zur Behandlung der Diarrhoe, dadurch gekennzeichnet, daß man eine Verbindung I, hergestellt nach Anspruch 1, mit pharmazeutisch geeigneten Hilfsstoffen mischt.

## Claims

**Claims for the following Contracting States: BE, CH, DE, FR, GB, IT, LI, LU, NL, SE**

1. A compound of the formula I

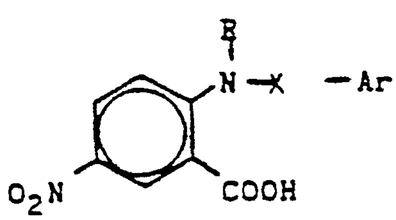

I

in which
   R represents hydrogen,
   X represents a chain $(CH_2)_n$, n being 1, 2, 3 or 4, which chain is unsubstituted or substituted by one to 2n $(C_1\text{-}C_2)$-alkyl radicals, it being additionally possible for the chain to be substituted by a radical Ar which is as defined below, and
   Ar represents an aromatic or heteroaromatic system which is unsubstituted or substituted by 1 to 3 identical or different groups $(C_1\text{-}C_2)$-alkyl, $(C_1\text{-}C_2)$-alkoxy, F, Cl, Br, I or $CF_3$
and its pharmaceutically tolerated salts.

2. A compound of the formula I as claimed in claim 1, wherein

X represents $(CH_2)_n$ with n = 2 or 3, and
Ar represents the unsubstituted phenyl radical.

**3.** A process for the preparation of a compound I as claimed in claim 1, which comprises
a) reaction of a compound of the formula II

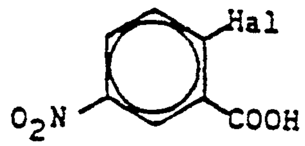

II

in which Hal represents F, Cl or Br, with amines of the formula

Ar-X-NHR

in which Ar, X and R have the said meanings,
b) reaction of a compound of the formula III

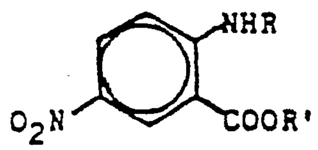

III

in which R is as defined above and in which R' represents $(C_1-C_4)$-alkyl, with a compound of the formula

Z-X-Ar

in which X and Ar are as defined above, and in which Z represents a leaving group, and elimination of the radical R',
c) reaction of a compound of the formula III

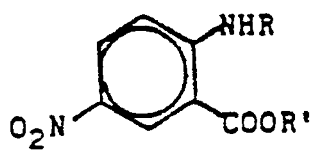

III

in which
R and R' have the abovementioned meanings, but R' additionally represents hydrogen, with compounds of the formula

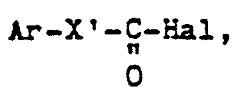

in which Ar has the abovementioned meaning, and X' represents a chain $(CH_2)_{n-1}$ which is unsubstituted or substituted by 1 to (2n-1) alkyl radicals having 1 to 2 carbon atoms or by an additional radical Ar, and
Hal represents Cl or Br,
to give a compound of the formula IV

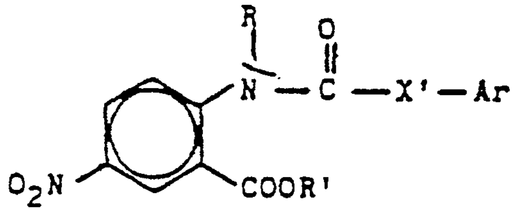

IV

and reduction of the latter to give a compound V

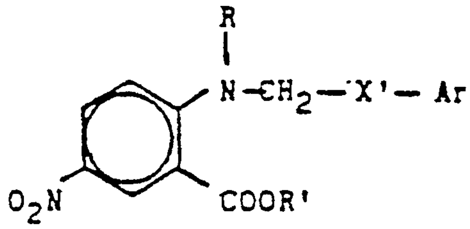

V

and elimination of the radical R'.

4. A medicament which contains an effective amount of at least one compound I as claimed in claim 1.

5. The use of a compound I as claimed in claim 1 for the preparation of a medicament for the treatment of cerebral edema.

6. The use of a compound I as claimed in claim 1 for the preparation of a medicament for the treatment of diarrhea.

**Claims for the following Contracting State: AT**

1. A process for the preparation of a compound I

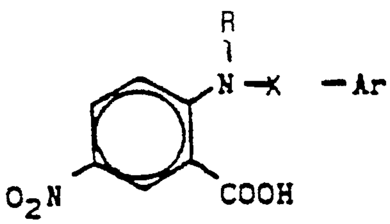

I

in which

R represents hydrogen,

X represents a chain $(CH_2)_n$, n being 1, 2, 3 or 4, which chain is unsubstituted or substituted by one to 2n $(C_1\text{-}C_2)$-alkyl radicals, it being additionally possible for the chain to be substituted by a radical Ar which is as defined below, and

Ar represents an aromatic or heteroaromatic system which is unsubstituted or substituted by 1 to 3 identical or different groups $(C_1\text{-}C_2)$-alkyl, $(C_1\text{-}C_2)$-alkoxy, F, Cl, Br, I or $CF_3$ and its pharmaceutically tolerated salts, which comprises

a) reaction of a compound of the formula II

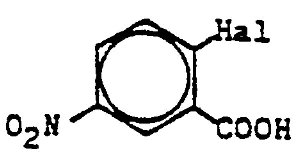

II

in which Hal represents F, Cl or Br, with amines of the formula

Ar-X-NHR

in which Ar, X and R have the said meanings,
b) reaction of a compound of the formula III

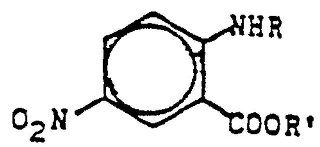

III

in which R is as defined above and in which R' represents ($C_1$-$C_4$)-alkyl, with a compound of the formula

Z-X-Ar

in which X and Ar are as defined above, and in which Z represents a leaving group, and elimination of the radical R',
c) reaction of a compound of the formula III

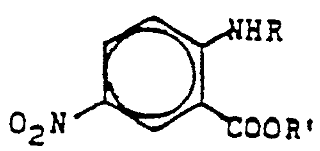

III

in which
R and R' have the abovementioned meanings, but R' additionally represents hydrogen, with compounds of the formula

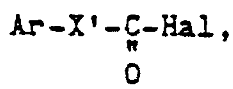

in which
Ar has the abovementioned meaning, and X' represents a chain $(CH_2)_{n-1}$ which is unsubstituted or substituted by 1 to (2n-1) alkyl radicals having 1 to 2 carbon atoms or by an additional radical Ar, and
Hal represents Cl or Br,
to give a compound of the formula IV

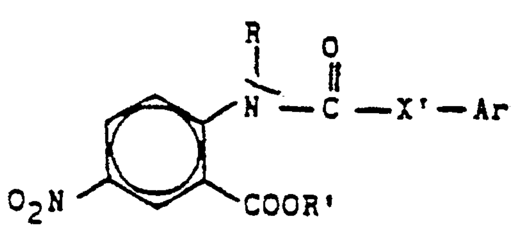

IV

and reduction of the latter to give a compound V

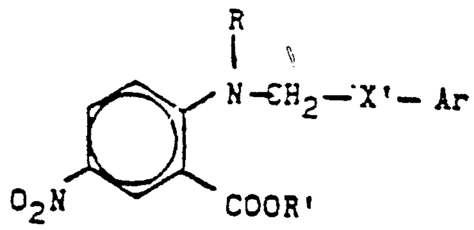

V

and elimination of the radical R'.

2. The process as claimed in claim 1, wherein X represents $(CH_2)_n$ with n = 2 or 3, and Ar represents the unsubstituted phenyl radical.

3. A process for the preparation of a medicament for the treatment of cerebral edema, which comprises a compound I, prepared as claimed in claim 1, being mixed with pharmaceutically suitable auxiliaries.

4. A process for the preparation of a medicament for the treatment of diarrhea, which comprises a compound I, prepared as claimed in claim 1, being mixed with pharmaceutically suitable auxiliaries.

**Revendications**

**Revendications pour les Etats contractants suivants: BE, CH, DE, FR, GB, IT, LI, LU, NL, SE**

1. Composés de formule I

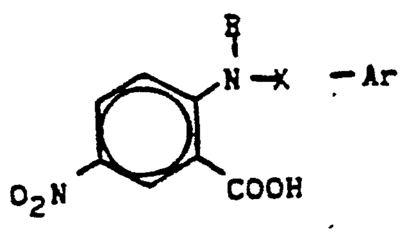

I

dans laquelle

R représente un atome d'hydrogène;

X représente une chaîne $(CH_2)_n$, n étant 1, 2, 3 ou 4, et laquelle chaîne n'est pas substituée ou est substituée par 1 à 2n radicaux alkyle en $C_1$-$C_2$, la chaîne pouvant en outre être substituée par un radical Ar qui est tel que défini ci-dessous;

Ar représente un système aromatique ou hétéroaromatique qui n'est pas substitué ou est substitué par 1 à 3 atomes ou groupes, identiques ou différents, alkyle en $C_1$-$C_2$, alcoxy en $C_1$-$C_2$, F, Cl, Br, I ou $CF_3$,

ainsi que leurs sels pharmaceutiquement acceptables.

2. Composé de formule I selon la revendication 1, caractérisé en ce que
X représente $(CH_2)_n$ avec n = 2 ou 3, et
Ar représente le radical phényle non substitué.

3. Procédé pour la préparation d'un composé I selon la revendication 1, caractérisé en ce que
a) on fait réagir un composé de formule II

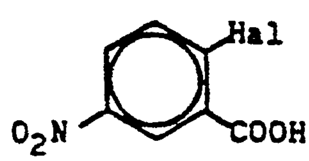

II

dans laquelle Hal représente F, Cl ou Br, avec des amines de formule

Ar-X-NHR,

dans laquelle Ar, X et R ont les significations données;
b) on fait réagir un composé de formule III

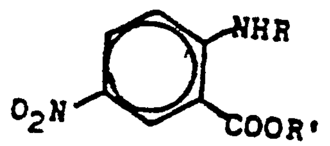

III

dans laquelle R est tel que défini plus haut et dans laquelle R' représente un groupe alkyle en $C_1$-$C_4$, avec un composé de formule

Z-X-Ar,

dans laquelle X et Ar sont tels que définis plus haut, et dans laquelle Z représente un groupe séparable,
et en ce qu'on élimine le radical R';
c) on fait réagir un composé de formule III

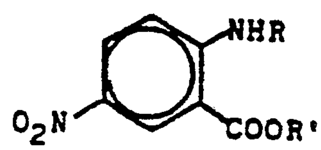

III

dans laquelle R et R' ont les significations données plus haut, mais R' représente en outre un atome d'hydrogène, avec des composés de formule

Ar-X'-C-Hal,
‖
O

dans laquelle Ar a la signification donnée plus haut et X' représente une chaîne $(CH_2)_{n-1}$ qui n'est pas substituée ou est substituée par 1 à (2n-1) radicaux alkyle ayant 1 ou 2 atomes de carbone, ou par un radical Ar supplémentaire,
et
Hal représente Cl ou Br;
pour aboutir à un composé de formule IV

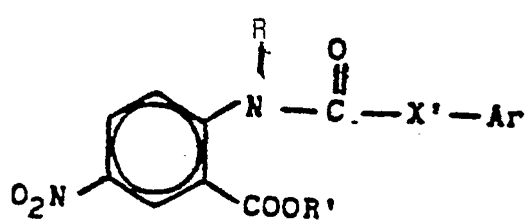

IV

et on réduit celui-ci pour aboutir à un composé V

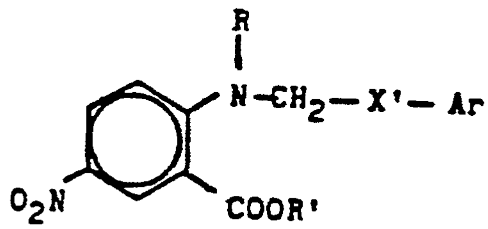

V

et on élimine le radical R'.

**4.** Médicament, caractérisé en ce qu'il contient une quantité efficace d'au moins un composé I selon la revendication 1.

**5.** Utilisation d'un composé L selon la revendication 1, pour la fabrication d'un médicament pour le traitement de l'oedème cérébral.

**6.** Utilisation d'un composé I selon la revendication 1, pour la fabrication d'un médicament pour la traitement de la diarrhée.

**Revendications pour l'Etat contractant suivant: AT**

**1.** Procédé pour la préparation d'un composé I

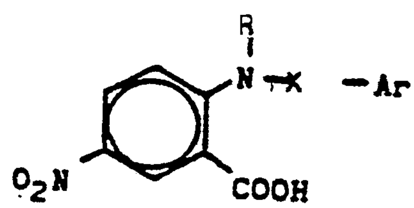

I

dans laquelle

R représente un atome d'hydrogène;

X représente une chaîne $(CH_2)_n$, n étant 1, 2, 3 ou 4, et laquelle chaîne n'est pas substituée ou est substituée par 1 à 2n radicaux alkyle en $C_1$-$C_2$, la chaîne pouvant en outre être substituée par un radical Ar qui est tel que défini ci-dessous;

Ar représente un système aromatique ou hétéroaromatique qui n'est pas substitué ou est substitué par 1 à 3 atomes ou groupes, identiques ou différents, alkyle en $C_1$-$C_2$, alcoxy en $C_1$-$C_2$, F, Cl, Br, I ou $CF_3$,

ainsi que de ses sels pharmaceutiquement acceptables, caractérisé en ce que

a) on fait réagir un composé de formule II

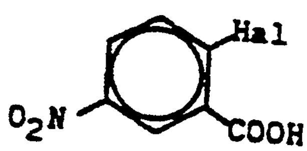

II

dans laquelle Hal représente F, Cl ou Br, avec des amines de formule

Ar-X-NHR,

dans laquelle Ar, X et R ont les significations données,

b) on fait réagir un composé de formule III

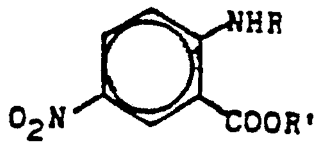

III

dans laquelle R est tel que défini plus haut et dans laquelle R' représente un groupe alkyle en $C_1$-$C_4$, avec un composé de formule

Z-X-Ar,

dans laquelle X et Ar sont tels que définis plus haut, et dans laquelle Z représente un groupe séparable,
et en ce qu'on élimine le radical R';
c) on fait réagir un composé de formule III

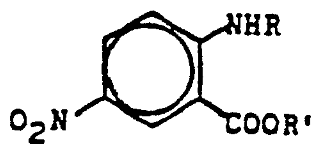

III

dans laquelle R et R' ont les significations données plus haut, mais R' représente en outre un atome d'hydrogène, avec des composés de formule

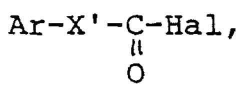

dans laquelle Ar a la signification donnée plus haut et X' représente une chaîne $(CH_2)_{n-1}$ qui n'est pas substituée ou est substituée par 1 à (2n-1) radicaux alkyle ayant 1 ou 2 atomes de carbone, ou par un radical Ar supplémentaire,
et
Hal représente Cl ou Br;
pour aboutir à un composé de formule IV

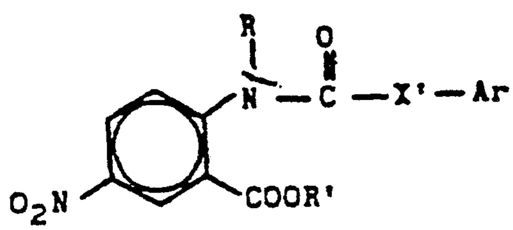

IV

et on réduit celui-ci pour aboutir à un composé V

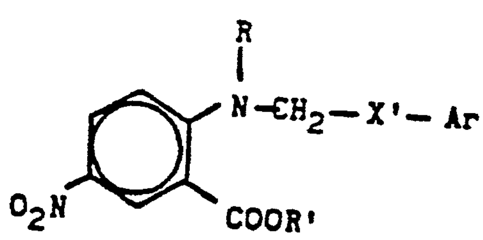

V

et on élimine le radical R'.

2. Procédé selon la revendication 1, caractérisé en ce que
   X représente $(CH_2)_n$ avec n = 2 ou 3, et
   Ar représente le radical phényle non substitué.

3. Procédé pour la fabrication d'un médicament pour le traitement de l'oedème cérébral, caractérisé en ce que l'on mélange avec des adjuvants pharmaceutiquement appropriés un composé I préparé selon la revendication 1.

4. Procédé pour la fabrication d'un médicament pour le traitement de la diarrhée, caractérisé en ce que l'on mélange avec des adjuvants pharmaceutiquement appropriés un composé I préparé selon la revendication 1.